# EUROPEAN PATENT SPECIFICATION

(11) **EP 2 176 327 B1**
(45) Date of publication and mention of the grant of the patent: **10.11.2010**
(21) Application number: 08774923.0
(22) Date of filing: 09.07.2008
(51) Int. Cl.: C08J 11/10, C07C 51/09

(54) **METHOD FOR THE CHEMICAL DEPOLYMERIZATION OF WASTE POLYETHYLENE TEREPHTHALATE**
VERFAHREN ZUR CHEMISCHEN DEPOLYMERISATION VON ALT-POLYETHYLENTEREPHTHALAT
PROCÉDÉ DE DÉPOLYMÉRISATION CHIMIQUE DE DÉCHETS TÉRÉPHTALATE DE POLYÉTHYLÈNE

(30) Priority: 13.07.2007 CZ 20070469
(43) Date of publication of application: 21.04.2010
(73) Proprietor: Ustav Chemickych Procesu Akademie Ved Ceské Republiky, 165 02 Praha 6 - Suchdol (CZ)
(72) Inventor: HAJEK, Milan, 160 00 Praha 6 (CZ); SOBEK, Jiri, 792 01 Bruntal (CZ); BRUSTMAN, Jaroslav, 471 14 Kamenicky Senov (CZ)
(74) Representative: Rezac, Petr
(86) International application number: PCT/EP2008/058917
(87) International publication number: WO 2009/010435

(56) References cited:
- US-A1- 2005 096 482
- ANDREJ KRZAN: "Poly(ethylene terephthalate) Glycolysis Under Microwave Irradiation" POLYMERS FOR ADVANCED TECHNOLOGIES, vol. 10, 1999, pages 603-606, XP002509810
- LIXIN LIU ET AL.: "Hydrolytic Depolymerization of Poly(ethylene terephthalate) Under Microwave Irradiation." JOURNAL OF APPLIED POLYMER SCIENCE, vol. 95, 2005, pages 719-723, XP002509811

## Description

### Technical field

The invention relates to a method for the chemical depolymerization of polyethylene terephthalate (PET), in particular in the form of PET bottles, by application of microwave radiation and solvolysis in the presence of a catalyst into appropriate monomers, i.e. terephthalic acid, or its derivatives, and ethylene glycol.

### Background art

The recycling of waste polyethylene terephthalate, especially PET bottles, is currently one of the most important tasks in the polymers recycling industry. The driving force behind the PET bottles recycling is the accumulated waste PET material produced by the beverage industry. At present, the majority of PET bottles are processed into fibres. Because of troubles in the use of waste PET bottles for the production of fresh PET bottles in so-called "bottle to bottle" technology and insufficient sorting the chemical processing of waste PET bottles into monomers is becoming more attractive. The chemical depolymerization, which is especially advantageous in the case of heavily contaminated PET material, is based on the solvolysis and includes most frequently hydrolysis, methanolysis, glycolysis, or potentially ammonolysis i.e. processes by which monomers that can be used for the synthesis of new PET products are formed.

These methods include hydrolysis using acids or bases in the aqueous medium, US 4,355,175, alcoholysis or glycolysis in the presence of various catalysts, Journal of Applied Polymer Science 34, 235 (1989), or potentially hydrolysis or alcoholysis in the supercritical medium, US 4,605,762. However, all these aforementioned methods require harsh reaction conditions such as the use of strong acids or bases, high temperature, and in particular, elevated pressure. Patents US 3,544,622, EP 597751, and Czech patents CZ 288622, CZ 296280 also deal with the chemical recycling of the waste PET material.

It is known that using microwave radiation as an energy source in chemical reactions increased rate of reaction, i.e. reduced reaction time and higher product yield may be achieved even under milder reaction conditions. Recently, a PET material depolymerization using for the alcoholysis (methanol, propylene glycol, polyethylene glycol) microwave radiation was described in the patent SI 9800060. The depolymerization was performed under elevated pressure in the presence of zinc acetate as a catalyst. Likely, Chinese patents CN 1401688 and CN 1594268 describe the PET material depolymerization in the presence of microwave radiation by using only water without catalyst, but under high pressure (20 bar) and temperature (220 - 230°C)

The PET material depolymerization by the effect of microwave radiation under atmospheric pressure was recently described by Iranian authors in the journal of Advances of Polymer Technology 25, 242-246 (2006). The paper describes hydroglycolysis carried out in excess of C₂ - C₆ alcohols in the presence of basic catalysts (sodium hydroxide, potassium hydroxide) and sodium and zinc acetate, respectively. However, the material subjected to the depolymerization reaction was a pure PET material, which was easy to get depolymerized even without any microwave radiation present (100°C, 30 min, 100% conversion) and not a waste PET. The Czech patent CZ 296343 describes the acidic hydrolysis of waste PET material by means of strong acids such as nitric acid or perchloric acid, or mixtures thereof, by the effect of microwave radiation. The hydrolysis was apparently carried out under elevated pressure but the ethylene glycol formed was completely destructed.

### Disclosure of Invention

The primary object of the invention is to provide a method for the chemical depolymerization of polyethylene terephthalate by application of microwave radiation and solvolysis in the presence of a catalyst. Specifically, in the first stage the waste polyethylene terephthalate is mixed up with an microwaves absorbing activator, the mixture is melted by its exposing to a microwave radiation on a frequency from 915 to 2450 MHz and with a power output from 0.1 to 0.5 kW per kg of a charge, at a temperature from 230 to 330 °C, under atmospheric pressure and in the second stage, the molten mixture is subjected to solvolysis, including acidic or basic hydrolysis, alcoholysis or glycolysis in the presence of a catalyst under continuing microwave radiation and atmospheric pressure yielding terephthalic acid, salts or esters thereof, and ethylene glycol.

Other aspects of the method according to the invention are apparent from the hereinafter described or detailed potential specific embodiments thereof.

As an activator silicon carbide, tungsten carbide, ferrite, magnetite, active carbon, or polar liquids as alcohols (methanol, ethanol) diols (ethylene glycol, propylene glycol), ketones (acetone, acetophenone), acids (p-toluene sulphonic acid, terephthalic acid, formic acid, or acetic acid), or water and their mixtures at the amount of 1 - 30 % by weight based on the PET raw material may be employed.

The acidic hydrolysis may be carried out in the presence of acidic catalysts, for instance heterogeneous catalysts as montmorillonites K10 and KSF, ion exchangers, zeolites, phosphoric acid supported on alumina or silica, furthermore, copper(II), iron(III), zinc(II), aluminium(III), antimony(III), bismuth(III) chlorides or acetates, respectively, or using homogeneous catalysts as p-toluene sulphonic, formic, acetic, benzoic, terephthalic , or sulphuric acid, respectively.

Alternatively, the alkaline hydrolysis may be carried out in the presence of strong bases such as alkaline metal hydroxide (sodium hydroxide, potassium or lithium hydroxide, respectively), or potentially in the presence of phase transfer catalysts, for example TOMAB (trioctyl methyl ammonium bromide).

The alcoholysis may be carried out in the presence of alcohol such as methanol or ethanol, or diol such as ethylene glycol and transesterification catalysts in particular zinc(II) or ferric(III) chloride, or manganese(II), cobalt(II), calcium(II), and magnesium (II) acetates, respectively.

The depolymerization may be carried out in the presence of ionic liquid being added in the second stage of the depolymerization.

In the second stage of the depolymerization process, the molten mixture may be exposed to a microwave radiation at a temperature between 100 and 220° C and in both the first and the second stage the depolymerization may be carried out in air or in inert atmosphere of nitrogen or argon, in either a batch or continuous process.

The melting process runs at high rate in the whole bulk volume and the shape of the feed material has no influence on the melting rate. Molten and partially depolymerised PET material may be transferred either directly into an solvolytic solution, where it undergoes hydrolysis or alcoholysis, or it may be left to solidify and be crushed or shredded into grain 0.1 to 2 mm in size prior to being transferred into the second stage.

The combination of microwave technology and the methods according to the invention, including application of an activator, presents very energy saving technique in order of magnitude from 30 to 50 % of the electric energy consumption compared with classic methods of the thermal depolymerization and provides valuable resulting products, which then can be directly used or be subject to further easy processing.

### Mode(s) for Carrying Out the Invention

Example 1

2 kg waste polyethylene terephthalate containing 7 wt % impurities is mixed with an activator represented by 10 % acetic acid in a 10:1 ratio. The resulting mixture is fed into a ceramic crucible provided with a bottom discharge valve and the crucible is placed into a microwave oven lined with insulating material permeable for microwave radiation. Then the mixture in the crucible is exposed to microwave radiation on a frequency of 2450 MHz and with a power output of 0.7 kW for the period of 30 minutes at a temperature of 250° C until the mixture material is molten. The molten and partially depolymerised PET material is then discharged into a solvolytic reactor containing water or a solution of alkaline hydroxide, or acid, and the depolymerization is completed in the microwave oven under microwave radiation in the presence of a solvolytic catalyst yielding ethylene glycol., terephthalic acid, or derivatives thereof. Ethylene glycol is then separated by distillation under reduced pressure and terephthalic acid and its derivatives are separated by conventional processes, i.e. by acid filtration and esters distillation.

Example 2

The process of melting waste PET material is conducted under the same conditions as in example 1 except that as the melting activator 20 wt % silicon carbide, tungsten carbide, ferrite, magnetite, active carbon or polar liquids as alcohols (methanol, ethanol), diols (ethylene glycol, propylene glycol), ketones (acetone, acetophenone), acids (p-toluene sulphonic, terephthalic , formic, and acetic acid, respectively), water and their mixtures is subsequently used. The melt is then withdrawn from the bottom outlet and continuously refilled through the upper inlet. The melt is added to a solvolytic solution or it can be left to solidify and after being crushed to grain 0.1 to 2.0 mm in size subjected to the depolymerization in the second stage.

Example 3

In the second stage, 20 g crushed PET material prepared according to example 2 is subjected to acidic hydrolysis in the mixture of 40 ml water and 10 g iron(III) chloride. The reaction mixture is exposed to microwave radiation in a microwave oven in the nitrogen atmosphere and maintained at its boiling point. 83 % conversion into terephthalic acid and ethylene glycol is achieved in 90 minutes.

Example 4

The acidic hydrolysis according to the example 3 is carried out in the second stage on the same conditions under the microwave radiation except that in the second stage iron(III) chloride is replaced subsequently with montmorillonite K10 and KSF, ion exchangers, zeolites, phosphoric acid supported on alumina or silica, furthermore, with chlorides or acetates of copper(II), iron(III), zinc(II), aluminium(III), antimony (III), bismuth(III), or homogeneous catalysts as p-toluene sulphonic acid, formic acid, acetic acid, benzoic acid, terephthalic acid, or sulphuric acid, and ionic liquids, respectively.

Example 5

20 g crushed waste PET material as in example 2 undergoes the alkaline hydrolysis in the mixture of 50 ml water and 10 g lithium hydroxide, or potentially sodium or potassium hydroxide and a phase transfer catalyst, for example TOMAB (trioctyl methyl ammonium bromide). At a temperature of 106° C, 91 % conversion into the corresponding salt of terephthalic acid and ethylene glycol is achieved in 60 minutes.

Example 6

20 g crushed waste PET material as in example 2 undergoes the alcoholysis in the presence of 20 ml methanol and 20 g zinc(II) chloride. At the boiling temperature of the reaction mixture, 95 % conversion into methyl ester of terephthalic acid and ethylene glycol is achieved within 60 minutes.

Example 7

The process of alcoholysis according to example 6 is carried out in the presence of ethanol or ethylene glycol under the same conditions with the exception that zinc(II) chloride is replaced by iron(III) chloride, or manganese(II), cobalt(II), calcium(II), and magnesium(II) acetate, respectively.

### Industrial Applicability

The invention can be used for the solution of the problems of accumulated waste, in particular waste based on PET bottles, by way of its total depolymerization into monomers, i.e. ethylene glycol and terephthalic acid, or derivatives thereof, and the reuse thereof for the PET bottle production.

## Claims

1. A method of the chemical depolymerization of waste polyethylene terephthalate by application of microwave radiation and solvolysis in the presence of a catalyst **characterized in that** in the first stage the waste polyethylene terephthalate is mixed up with an microwaves absorbing activator, the mixture is melted by its exposing to a microwave radiation on a frequency from 915 to 2450 MHz and with a power output from 0.1 to 0.5 kW per kg of a charge, at a temperature from 230 to 330° C, under atmospheric pressure and in the second stage, the molten mixture is subjected to solvolysis, including acidic or basic hydrolysis, alcoholysis or glycolysis in the presence of a catalyst and under continuing microwave radiation and atmospheric pressure yielding terephthalic acid, salts or esters thereof, and ethylene glycol.

2. The method according to claim 1 **characterized in that** the activator is selected from the group consisting of silicon carbide, tungsten carbide, ferrite, magnetite, active carbon, or polar liquids as alcohols (methanol, ethanol) diols (ethylene glycol, propylene glycol), ketones (acetone, acetophenone), acids (p-toluene sulphonic acid, terephthalic acid, formic acid, or acetic acid), or water, and their mixtures, the amount of the activator being 1-30 % by weight based on the PET raw material.

3. The method according to claim 1 or 2 **characterized in that** the acidic hydrolysis is carried out in the presence of acidic catalysts selected from the group consisting of heterogeneous catalysts such as montmorillonites K10 and KSF, ion exchangers, zeolites, phosphoric acid supported by a carrier such as alumina or silica, furthermore, copper(II), iron(III), zinc(II), aluminium(III), antimony(III), bismuth(III) chloride or acetate, respectively, or homogeneous catalysts such as p-toluene sulphonic, formic, acetic, benzoic, terephthalic , or sulphuric acid.

4. The method according to claim 1 or 2 **characterized in that** the alkaline hydrolysis is carried out in the presence of strong bases such as alkaline metal hydroxides (sodium hydroxide, potassium or lithium hydroxide, respectively), or in the presence of phase transfer catalysts, such as TOMAB (trioctyl methyl ammonium bromide).

5. The method according to claim 1 or 2 **characterized in that** the alcoholysis is carried out in the presence of an alcohol such as methanol, ethanol, ethylene glycol and trans-esterification catalysts selected from the group consisting of zinc(II) or iron(III) chloride, respectively, or manganese(II), cobalt(II), calcium(II), and magnesium(II) acetates.,

6. The method according to any of the claims 1 to 5 **characterized in that** the depolymerization is carried out in the presence of ionic liquid added to the molten mixture in the second stage of depolymerization.

7. The method according to any of claims 1 to 5 **characterized in that** that in first and the second stage the depolymerization is carried out in air or an inert atmosphere of nitrogen or argon.

8. The method according to any of the claims 1 to 7 **characterized in that** in the second stage of the depolymerization process the molten mixture is exposed to a microwave radiation at the temperature between 100 and 220° C.

9. The method according to any of claims 1 to 8 **characterized in that** in the first and the second stage the depolymerization is carried out in a batch or continuous process.

## Patentansprüche

1. Verfahren zur chemischen Depolymerisation von Polyethylenterephthalat - Abfällen unter Wirkung einer Mikrowellenstrahlung und Verwendung der Solvolyse und in Anwesenheit eines Katalysators **dadurch gekennzeichnet, dass** in der ersten Stufe Polyethylenterephthalat-Abfälle mit einem die Mikrowellen absorbierten Aktivator vermischt wird, das Gemisch durch Aussetzung einer Mikrowellenstrahlung mit Frequenz von 915 bis 2450 MHz und Leistung des Strahlers von 0,1 bis 0,5 kW/kg des Einsatzes, bei einer Temperatur von 230 bis 330 °C und bei atmosphärischem Druck zerschmolzen wird und in der zweiten Stufe in Anwesenheit eines Katalysators und unter fortlaufender Mikrowellenstrahlung und atmosphärischem Druck einer alkalischen Glykolyse, Hydrolyse, Alkoholyse, oder Glykolyse umfassenden Solvolyse unterworfen wird, wobei Terephthalatsäure, oder Salze oder Ester von Terephthalatsäure und Äthylenglykol zurück gewonnen werden.

2. Verfahren nach Anspruch 1 **dadurch gekennzeichnet, dass** der Aktivator aus einer aus Silikonkarbid, Wolframkarbid, Ferit, Magnetit, aktives Kohlenstoff oder polare Flüssigkeiten, wie Alkoholen ( Methanol, Äthanol), Diolen (Äthylenglykol, Propylenglykol) , Ketonen (Azeton, Azetophenon), Säuren (p-Toluolsulfonsäure, Terephthalatsäure, Ameisensäure oder Essigsäure) oder Wasser und ihre Gemische, bestehenden Gruppe ausgewählt ist, wobei die Menge des Aktivators 1 - 30 Massenprozent, bezogen auf das Rohstoff-PET-Material, beträgt.

3. Verfahren nach Anspruch 1 oder 2 **dadurch gekennzeichnet, dass** die säurehaltige Hydrolyse in Anwesenheit von säurehaltigen Katalysatoren durchgeführt wird, die aus einer aus heterogenen Katalysatoren, wie Montmorilloniten K10 und KSF, Ionenaustauscher, Zeolithe, Phosphorsäure bestehenden Gruppe ausgewählt sind, wobei die heterogenen Katalysatoren auf einem Träger, wie Aluminium oder Kieselerde, und weiter auf Kupfer (II)-, Eisen (III)-, Zink (II)-, Aluminium (III)-, Antimon (III)-, Wismut (lll)- Chloriden oder Azetaten fixiert sind, oder aus einer aus homogenen Katalysatoren, wie p-Toluolsulfon-, Ameisen-, Essig-, Benzoisch-, Terephthalat- oder Schwefelsäure bestehenden Gruppe ausgewählt sind.

4. Verfahren nach Anspruch 1 oder 2 **dadurch gekennzeichnet, dass** die alkalische Hydrolyse in Anwesenheit von starken Basen, wie alkalische Metallhydroxide (Natriumhydroxid, Kalium- oder Lithiumhydroxid) oder in Anwesenheit von phasenübertragenden Katalysatoren, wie TOMAB (Trioktyl- Methyl- Ammoniumbromid) durchgeführt wird.

5. Verfahren nach Anspruch 1 oder 2 **dadurch gekennzeichnet, dass** die Alkoholyse in Anwesenheit eines Alkohols, wie Methanol, Äthanol, Äthylenglykol und Übertragung-Veresterungskatalysatoren durchgeführt wird, die aus einer aus Zink(II) oder Eisen(III)- Chloriden oder Mangan(II), Kobalt(II), Kalzium(II), Magnesium(II)-Azetaten bestehenden Gruppe ausgewählt sind.

6. Das Verfahren nach einem der Ansprüche 1 bis 5 **dadurch gekennzeichnet, dass** die Depolymerisation in Anwesenheit einer ionischen Flüssigkeit durchgeführt wird, die in der zweiten Stufe der Depolymerisation zu dem geschmolzenen Gemisch zugesetzt wird.

7. Verfahren nach einem der Ansprüche 1 bis 5, **dadurch gekennzeichnet, dass** in der ersten und zweiten Stufe die Depolymerisation in einer Luft- oder inerter Stickstoff- oder Argonatmosphäre durchgeführt wird.

8. Das Verfahren nach einem der Ansprüche 1 bis 7 **dadurch gekennzeichnet, dass** in der zweiten Stufe des Depolymerisationsvorgang das geschmolzene Gemisch einer Mikrowellenstrahlung bei der Temperatur zwischen 100 und 220 °C ausgesetzt wird.

9. Verfahren nach einem der Ansprüche 1 bis 8 **dadurch gekennzeichnet, dass** in der ersten und zweiten Stufe die Depolymerisation in einem Stapel- oder kontinuierlichen Prozess durchgeführt wird.

## Revendications

1. Un procédé d'une depolymérisation chimique d'un polyéthylentéréphtalate résiduel par action d'une radiation micro-onde et utilisant une solvolyse en présence d'un catalyseur, **caractérisé en ce que** le polyéthylentéréphtalate résiduel est mélangé, dans la première étape, avec un activateur absorbant des micro-ondes, le mélange est porté à fusion par exposition à une radiation micro-onde de fréquence de 915 à 2450 MHz à une puissance de radiateur de 0,1 à 0,5 kW/kg de la charge, à une température de 230 à 330 °C et à la pression atmosphérique et soumis, dans la deuxième étape, à la solvolyse, renfermant hydrolyse acide ou alcaline, alcoolyse ou glycolyse, en présence du catalyseur et sous l'action de la radiation micro-onde continuée et à la pression atmosphérique en obtenant acide téréphtalique, ses sels ou ses esters et glycol éthylénique.

2. Le procédé selon la revendication 1, **caractérisé en ce que** l'activateur est choisi d'un groupe comprenant carbure de silicium, carbure de tungstène, ferrite, magnétite, carbone actif ou liquides polaires, tels qu'alcools (méthanol, éthanol), diols (glycol éthylénique, glycol propylénique), cétones (acétone, acétophénone), acides (acide p-toluènesulphonique, téréphtalique, formique ou acétique) ou l'eau et leurs mélanges, la quantité de l'activateur étant 1 - 30 % de poids, rapportée au matériau brut PET.

3. Le procéde selon les revendications 1 ou 2, **caractérisé en ce que** l'hydrolyse acide est réalisée en présence de catalyseurs acides choisis d'un groupe renfermant soit des catalyseurs hétérogènes, tels que montmorillonites K10 et KSF, des échangeurs d'ions, zéolites, acide phosphorique déposé sur un support, tel qu'alumine ou silice, ou aussi chlorure ou acétate de cuivre(II), de fer(III), de zinc(II), d'aluminium(III), d'antimoine(III), de bismuth(III), soit des catalyseurs homogènes, tels qu'acide p-toluènesulphonique, acide formique, acide acétique, acide benzoïque, acide téréphtalique ou acide sulfurique.

4. Le procédé selon les revendications 1 ou 2, **caractérisé en ce que** l'hydrolyse alcaline est réalisée en présence de bases fortes, telles qu'hydroxydes alcalins (hydroxyde de sodium, hydrixyde de potassium ou hydroxyde de lithium), éventuellement en présence de catalyseurs du transport de phases, tels que TOMAB (trioctylméthylammoniumbromure), par exemple.

5. Le procédé selon les revendications 1 ou 2, **caractérisé en ce que** l'alcoolyse est réalisée à un milieu d'alcool, tel que méthanol, éthanol, glycol éthylénique, et de catalyseurs de transestérification choisis d'un groupe renfermant chlorure de zinc(II) ou de fer(III) ou acétate de manganèse(II), de cobalt(II), de calcium(II) et de magnésium(II).

6. Le procédé selon l'une quelconque des revendications 1 à 5, **caractérisé en ce que** la depolymérisation est réalisée en présence d'un liquide ionique qui est ajoutée dans le mélange fondu à la deuxième étape de la depolymérisation.

7. Le procédé selon l'une quelconque des revendications 1 à 5, **caractérisé en ce que** la depolymérisation est réalisée, dans la première étape et la deuxième étape, soit à l'air soit sous une atmosphère inerte d'azote ou d'argon.

8. Le procédé selon l'une quelconque des revendications 1 à 7, **caractérisé en ce que** le mélange fondu est exposé, dans la deuxième étape de depolymérisation, à la radiation micro-onde à une température de 100 à 220 °C.

9. Le procédé selon l'une quelconque des revendications 1 à 8, **caractérisé en ce que** la depolymérisation est réalisée, dans la première étape et la deuxième étape, de manière continue ou de charge.
